(19) Europäisches Patentamt
European Patent Office
Office européen des brevets

(11) EP 1 637 133 A2

(12) **DEMANDE DE BREVET EUROPEEN**

(43) Date de publication:
**22.03.2006 Bulletin 2006/12**

(51) Int Cl.:
***A61K 31/185*** *(2006.01)* ***A61P 9/00*** *(2006.01)*

(21) Numéro de dépôt: **05112614.2**

(22) Date de dépôt: **03.04.1997**

(84) Etats contractants désignés:
**AT BE CH DE DK FI FR GB GR IE IT LI LU MC NL PT SE**

(30) Priorité: **03.04.1996 FR 9604182**

(62) Numéro(s) de document de la (des) demande(s) initiale(s) en application de l'article 76 CBE:
**97920619.0 / 0 954 298**

(71) Demandeur: **LABORATORIOS DEL DR. ESTEVE, S.A.**
**08026 Barcelona (ES)**

(72) Inventeur: **Esteve-Soler, José**
**E-08017, Barcelone (ES)**

(74) Mandataire: **Warcoin, Jacques et al**
**Cabinet Régimbeau**
**20, rue de Chazelles**
**75847 Paris cedex 17 (FR)**

Remarques:
Cette demande a été déposée le 21 - 12 - 2005 comme demande divisionnaire de la demande mentionnée sous le code INID 62.

(54) **Utilisation des dérives 2,5-dihydroxybenzènesulfoniques pour la fabrication de médicaments destinés à la normalisation de la fonction endothéliale pour le traitement des complications vasculaires du diabète, ainsi que des troubles vasculaires d'origine endothéliale**

(57) Utilisation des dérivés de 2,5-dihydroxybenzènesulfoniques de formule générale (I)

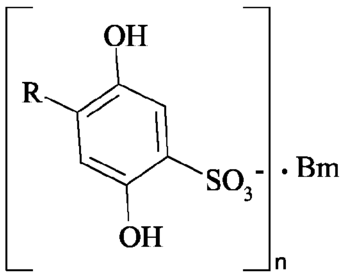

dans laquelle: R représente H ou $SO_3$-; B représente $Ca^{++}$ ou $H_2N(C_2H_5)_2$; n représente 1 ou 2; m représente 1 ou 2, pour la fabrication de médicaments destinés au traitement des complications vasculaires du diabète et des troubles vasculaires d'origine endothéliale.

EP 1 637 133 A2

## Description

La présente invention concerne l'utilisation des dérivés des acides dihydroxybenzènesulfoniques ainsi que leurs sels physiologiquement acceptables, pour la fabrication de médicaments destinés à la normalisation de la fonction endothéliale, au traitement de la dysfonction sexuelle, des complications vasculaires du diabète et des troubles vasculaires d'origine endothéliale.

Des études récentes ont démontré que le Dobésilate de calcium, l'Ethamsylate et le Persilate exercent des effets au niveau de l'Endothélium dans le sens de faciliter le relâchement vasculaire endothélium-dépendant. Cet effet s'observe tant chez les animaux normaux comme chez ceux à qui on a produit expérimentalement un vieillissement vasculaire moyennant l'administration de doses élevées de vitamine $D_2$. Cette activité du Dobésilate de calcium, de l'Ethamsylate et du Persilate peut se traduire chez l'homme par des effets d'utilité thérapeutique. En particulier, on a démontré que l'érection du pénis est modulée par l'oxyde nitrique produit au niveau endothélial (A.L Burnett et al., Science, 1992, 257: 401-403 ; J. Rajfer et al., N. Engl. J. Med., 1992, 326: 90-94; Editorial, Lancet, 1992, 340: 882-883) et que dans les circonstances où la fonction endothéliale est altérée, comme dans les hyperlipidémies (K. Kugiyama et al., Nature, 1990, 344: 160-162), la correction de l'altération normalise la fonction érectile, mesurée avec précision pendant le sommeil nocturne (J.-B. Kostis et al., J. Clin. Pharmacol., 1994, 34: 989-996; R.C. Rosen, "The Pharmacology of Sexual Function and Dysfunction", J. Bancroft ed. Elsevier Sc, 1995, pages 277-287). La normalisation de la fonction endothéliale obtenue avec le Dobésilate de calcium, l'Ethamsylate et le Persilate peut représenter une approche thérapeutique totalement nouvelle au problème de l'impuissance (I. Saenz de Tejada et al., N. Engl. J. Med., 1989, 320: 1025-1030) tant chez les patients avec des troubles vasculaires de causes diverses (diabète, artério-sclérose, etc) comme chez les patients où on ne peut détecter qu'un trouble fonctionnel.

D'autre part, l'effet normalisateur de la fonction Endothéliale peut offrir également des opportunités thérapeutiques dans des processus de spasmes vasculaires, dans les complications du diabète (W. Durante et al., Br. J. Pharmacol., 1988, 94: 463-468) et dans tous les troubles, l'éjaculation précoce inclue (E M. Hull et al., Neuropharmacology, 1994, 33: 1499-1504), où un déficit de formation de l'oxyde nitrique pour l'endothélium vasculaire apparaît évident.

Les composés préconisés dans le cadre de la présente invention répondent à la formule générale I :

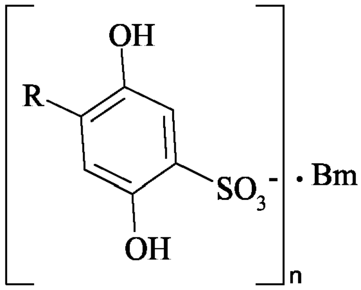

dans laquelle :

- R représente un atome d'hydrogène (H) on un groupement sulfonique ($S0_3^-$)
- B représente un atome de calcium ($Ca^{++}$) ou un groupement diéthylamine

$$[H_2N^+ (C_2H_5)_2]$$

- n représente 1 ou 2
- m représente 1 ou 2

Les composés des exemples que l'on indique ci-après se préparent d'après des procédés décrits antérieurement:

**Exemple 1:**

2,5-Dihydroxybenzène sulfonate de calcium (Dobésilate de calcium). "The Merck Index", 11th edition, Merck & Co., Rahway, N.J., USA, 1989.

**Exemple 2:**

2,5-Dihydroxybenzène sulfonate de diéthylamine (Ethamsilate). "The Merck Index", 11th edition, Merck & Co., R Rahway, N.J., USA, 1989.

**Exemple 3:**

2,5-Dihydroxybenzène-1,4-disulfonate de bis (diéthylamine) (Persilate de bis (diéthylamine). Brevet français FR 73/17709 (numéro de publication 2.201.888).

Pour étudier l'effet normalisateur de la fonction endothéliale des produits objet de la présente invention, on a réalisé différentes études "in vitro" et "in vivo". On décrit ci-après les résultats obtenus, à titre d'exemple non limitatif, avec un des produits objet de la présente invention, le Dobésilate de calcium, en démontrant qu'il possède une activité relâchante dépendant de l'Endothélium.

Les études ont été réalisées en utilisant les aortes isolées de lapins mâles, d'après les techniques décrites par A. Quintana et al. (Europ. J. Pharmacol, 1978, 53: 113-116) et par le groupe de l'Université d'Edimburg (Pharmacological Experiments on Isolated Preparations, Edimburg, Livingstone, 1970). Les aortes isolées sans l'Endothélium se préparent d'après la technique décrite par R. Furchgott et al. (J. Cardiol. Pharmacol., 1984, 6: S336-S343).

**A. ETUDES "IN VITRO"**

1) Effet sur la contraction de l'aorte, maintenu par $10^{-6}$M de noradrénaline.

Le Dobésilate de calcium relâche la contraction de noradrénaline de façon dépendante de la concentration (entre $10^{-4}$ M et $10^{-11}$ M).
Le relâchement maximum a été de 70 %.

2) Effet sur la tension basale dans les artères aortes avec et sans Endothélium (tension basale = 2 grammes).

Le Dobésilate de calcium à des concentrations entre $10^{-8}$ M et $10^{-4}$, relâche de façon dépendante de la dose les aortes soumises à une tension basale de 2 grammes. Le relâchement maximum a été de 44 % pour les artères avec de l'Endothélium et de 48 % pour les artères sans Endothélium.

3) Effet sur la courbe concentration-effet de noradrénaline.

Dans des artères avec l'Endothélium intact, le Dobésilate de calcium à la concentration de $10^{-6}$ M, inhibe un 44 % la contraction obtenue avec $10^{-4}$ M de noradrénaline. Par contre, avec des artères sans Endothélium, il n'y a aucune modification de la courbe.

**B. ETUDES "IN VIVO"**

On a étudié l'effet protecteur de l'Endothélium par le Dobésilate de calcium chez le lapin.

Pour cela on endommage l'Endothélium artériel chez les lapins par un traitement journalier avec une surdose de vitamine $D_2$ et on travaille avec trois groupes d'animaux :

O- Contrôle
I - Hypervitaminose $D_2$
II - Hypervitaminose $D_2$ + Dobésilate de calcium 50 mg/kg/jour.

On effectue les études avec les artères provenant des lapins traités en mesurant l'effet sur la contraction de l'aorte maintenu par $10^{-6}$ M de noradrénaline.

Le Dobésilate de calcium (entre $10^{-11}$ M et $10^{-5}$ M) relâche de façon dose-dépendante la contraction due à la noradrénaline. Le relâchement moindre correspond au groupe d'hypervitaminose $D_2$ (groupe I) alors que le relâchement maximum s'obtient avec les aortes du groupe traite avec le Dobésilate de calcium (groupe II).

Le relâchement maximum obtenu pour chaque groupe a été le suivant :

Groupe O (contrôle) : 69 %
Groupe I (hypervitaminose $D_2$ : 52 %M
Groupe II (hypervitaminose $D_2$ + Dobésilate de Calcium) : 100 %

Les résultats obtenus démontrent que le Dobésilate de calcium agit en potentialisant la synthèse et/ou la libération ou bien en diminuant la destruction d'un facteur relâchant qui dépend de l'Endothélium vasculaire et qui est probablement libéré par la noradrénaline à travers son action sur les réceptions $\alpha_2$ adrénergiques.

En thérapeutique humaine, la dose d'administration est bien sûr fonction de la gravité de affection à traiter. Elle sera généralement comprise entre environ 0,5 et environ 2 g/jour. Les dérivés de l'invention seront, par exemple, administrés sous forme de gélules ou de comprimés. On indiquera ci-après, à titre d'exemples, deux formes galéniques particulières.

Exemple de formule par gélule :

| | |
|---|---|
| Dobésilate de calcium | 0,500 g |
| Cellulose | 0,023 g |
| Estéarate de magnésium | 0,007 g |
| Dioxyde de silice colloïdale | 0,005 g |
| | 0,535 g |

Exemple de formule par comprimé :

| | |
|---|---|
| Dobésilate de calcium | 0,2500 g |
| Amidon de maïs | 0,0650 g |
| Lactose | 0,0520 g |
| Povidone K-30 | 0,0175 g |
| Acide citrique monohydrate | 0,0125 g |
| Estéarate de magnésium | 0,0020 g |
| Bisulfite de sodium | 0.0010 g |
| | 0,4000 g |

Compte tenu des intéressantes propriétés pharmacologiques attachées aux composés de formule générale I, la présente invention s'étend à l'application de ces composés à titre de médicaments, aux compositions pharmaceutiques les contenant et à leur utilisation pour la fabrication de médicaments destinés à la normalisation de la fonction endothéliale, au traitement de la dysfonction sexuelle, des complications vasculaires du

diabète et des troubles vasculaires d'origine endothéliale.

**Revendications**

**1.** Utilisation des dérivés de 2,5-dihydroxybenzènesulfoniques de formule générale 1 :

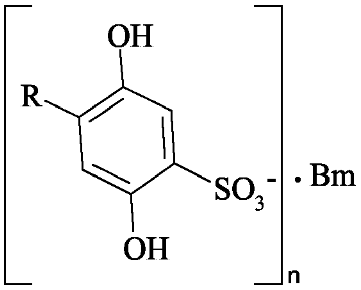

dans laquelle :

R représente H ou $SO_3^-$
B représente $Ca^{++}$ ou $H_2N^+(C_2H_5)_2$
n représente 1 ou 2
m représente 1 ou 2

pour la fabrication de médicaments destinés au traitement des complications vasculaires du diabète et des troubles vasculaires d'origine endothéliale.

**2.** Utilisation selon la revendication 1, **caractérisée en ce que** le dérivé de formule générale 1 est le 2,5-dihydroxybenzènesulfonate de calcium [DOBESILATE DE CALCIUM].

**3.** Utilisation selon la revendication 1, **caractérisée en ce que** le dérivé de formule générale I est le 2,5-dihydroxybenzènesulfonate de diéthylamine [ETHAMSYLATE] .

**4.** Utilisation selon la revendication 1, **caractérisée en ce que** le dérivé de formule générale 1 est le 2,5-dihydroxybenzène-1,4-disulfonate de bis (diéthylamine) [PERSILATE].